# EUROPEAN PATENT APPLICATION

(11) **EP 1 655 052 A2**
(43) Date of publication of application: **10.05.2006**
(21) Application number: 05024084.5
(22) Date of filing: 04.11.2005
(51) Int. Cl.: A61M 16/06, A61M 16/16, A61M 16/00

(54) **Wearable system for positive airway pressure therapy**

(30) Priority: 05.11.2004 US 982958
(71) Applicant: Air Products and Chemicals, Inc., Allentown, PA 18195-1501 (US)
(72) Inventor: Occhialini, James Michael, New Tripoli, PA 18066 (US); Yankovoy, Michael S., Wescosville, PA 18106 (US); Whitley, Roger Dean, Allentown, PA 18104-8501 (US); Graham, David Ross, Harleysville, PA 19438 (US)
(74) Representative: Kador & Partner

(57) **Abstract**

Apparatus (1) for providing positive airway pressure therapy comprising a mask (3) adapted for the delivery of pressurized air to maintain positive airway pressure in a patient's airway and an air pump system (5) comprising a plurality of pump elements adapted to supply pressurized air to the mask, wherein the air pump system (5) is attached directly to the mask (3) or is adapted to be worn on a part of the patient's body spaced apart from the mask (3).

## Description

### BACKGROUND OF THE INVENTION

Obstructive sleep apnea (OSA) is a breathing disorder suffered by an estimated 12 million people in the United States. This disorder is caused by a blockage of the airway, usually when the soft tissue in the rear of the throat collapses and closes during sleep. This causes interruptions in breathing and results in serious sleep deprivation, which in turn adversely affects the daily activities of individuals suffering from this disorder. The most common and effective treatment for sleep apnea is continuous positive airway pressure (CPAP) therapy. In this procedure, a patient wears a mask over the nose during sleep, and pressure from an air blower forces air through the nasal passages at prescribed pressures up to 25 cm water to prevent the patient's airway from collapsing. Some patients may use nasal prongs instead of a nasal mask, while other patients may require a full face mask covering both the nose and mouth.

Variations of CPAP therapy are available to improve patient treatment and comfort. In one variation, a higher pressure is applied during inhalation and a lower pressure is applied during exhalation. Alternatively, the pressure may be varied continuously in response to the patient's breathing pattern. In any of these options, the air pressure may be ramped up slowly over a period of time while the patient is falling asleep.

Numerous devices are commercially available to provide positive airway pressure therapy for sleep apnea patients. These airway pressure therapy devices use a base unit housing an air blower and air delivery control system, and may include air filtration, air heating, and/or humidification systems. Compliance and performance data logging options are also available. The base unit is placed at the patient's bedside and a flexible air hose connects the base unit with the patient's mask to deliver pressurized air at the proper pressure and flow rate. The flexible hose can be awkward and may limit a patient's ability to move about during sleep. Discomfort in the use of airway pressure devices can lead to dissatisfaction by patients and may cause reduced compliance in the use of the devices.

New positive airway pressure therapy devices are needed to provide improved sleeping comfort for sleep apnea patients and to increase compliance in the use of the devices by these patients. This need is addressed by embodiments of the invention described below and defined by the claims that follow.

### BRIEF SUMMARY OF THE INVENTION

One embodiment of the invention relates to an apparatus for providing positive airway pressure therapy comprising a mask adapted for the delivery of pressurized air to maintain positive airway pressure in a patient's airway and an air pump system including a plurality of pump elements adapted to supply pressurized air to the mask, wherein the air pump system is attached directly to the mask or is adapted to be worn on a part of the patient's body spaced apart from the mask.

The plurality of pump elements may be selected from the group consisting of piezoelectric pumps, thermopneumatic pumps, ultrasonically-driven pumps, electrostatic pumps, electro-osmosis pumps, electrohydrodynamic pumps, electromagnetic pumps, rotary pumps, shape memory alloy pumps, bimetallic pumps, diaphragm pumps, rotary vane pumps, scroll pumps, solenoid pumps, stepper-motor actuated pumps, piston pumps, and linear pumps. When the pump elements are diaphragm pumps, they may be adapted for parallel operation, series operation, or both parallel and series operation. The diaphragm pumps may be electrostatically activated or piezoelectrically activated.

The apparatus may further comprise a rechargeable power supply unit wearable by the patient and adapted to supply power to drive the air pump system, wherein the rechargeable power supply unit may comprise one or more batteries; alternatively, the rechargeable power supply unit may comprise one or more fuel cells.

The apparatus may further comprise a rechargeable power supply unit wearable by the patient and adapted to supply power to drive the air pump system and a base unit adapted to couple with the apparatus for providing positive airway pressure when the apparatus is not in use by the patient, wherein the base unit may include a recharging system adapted to recharge the power supply unit. The apparatus also may include a data monitoring and storage system to monitor and store operating data when the apparatus is in use by the patient. The base unit may include a data logging system adapted to download and store the data from the apparatus for providing positive airway pressure while the base unit is coupled with the apparatus for providing positive airway pressure.

The apparatus may further comprise a data monitoring and wireless transmission system adapted to monitor and transmit operating data while the apparatus is in use by the patient and the base unit may include a receiver adapted to receive and store the data transmitted by the wireless transmission system.

The apparatus may further comprise any of (1) a humidification system adapted to humidify inlet air to the air pump system or the pressurized air supplied to the mask; (2) a heating system adapted to heat inlet air to the air pump system or the pressurized air supplied to the mask; and (3) a filtration system adapted to filter inlet air to the air pump system or the pressurized air supplied to the mask.

The air pump system may be attached to and mounted on the mask to form an integrated mask and air pump system. The apparatus may further comprise a rechargeable power supply unit wearable by the patient and adapted to supply power to drive the air pump system and an electric supply wire to provide power from the power supply unit to the air pump system. The integrated mask and air pump system may comprise one or more straps adapted to secure the mask to the patient's face, and the rechargeable power supply may be mounted on at least one of the one or more straps. Alternatively, the rechargeable power supply unit may be mounted on an arm pack adapted to be worn on the patient's arm, a cap or headpiece adapted to be worn on the patient's head, a vest adapted to be worn on the patient's torso, or a waist pack adapted to be worn around the patient's waist. Alternatively, instead of a rechargeable power supply unit, the apparatus may include an electrical supply line attached at one end to the air pump system for supplying electric power thereto and an electrical plug at the other end for insertion into an external power outlet.

The apparatus for providing positive airway pressure therapy may further comprise a cap or headpiece adapted to be worn on the patient's head, wherein the air pump system is mounted on the cap or headpiece; a rechargeable power supply unit wearable by the patient and adapted to supply power to drive the air pump system, wherein the rechargeable power supply unit is mounted on the cap or headpiece; and a hose adapted to carry air from the air pump system to the mask. Alternatively, instead of a rechargeable power supply unit, the apparatus may include an electrical supply line attached at one end to the air pump system for supplying electric power thereto and an electrical plug at the other end for insertion into an external power outlet.

In another embodiment, the apparatus for providing positive airway pressure therapy may further comprise (c) a vest adapted to be worn about the patient's torso, wherein the air pump system is mounted on the vest; (d) a rechargeable power supply unit wearable by the patient and adapted to supply power to drive the air pump system, wherein the rechargeable power supply unit is mounted on the vest; and (e) a hose adapted to carry air from the air pump system to the mask. Alternatively, instead of a rechargeable power supply unit, the apparatus may include an electrical supply line attached at one end to the air pump system for supplying electric power thereto and an electrical plug at the other end for insertion into an external power outlet.

In an alternative embodiment, the apparatus for providing positive airway pressure therapy may further comprise (c) a waist pack adapted to be worn about the patient's waist, wherein the air pump system is mounted on the waist pack; (d) a rechargeable power supply unit wearable by the patient and adapted to supply power to drive the air pump system, wherein the rechargeable power supply unit is mounted on the waist pack; and (e) a hose adapted to carry air from the air pump system to the mask. Alternatively, instead of a rechargeable power supply unit, the apparatus may include an electrical supply line attached at one end to the air pump system for supplying electric power thereto and an electrical plug at the other end for insertion into an external power outlet.

In another alternative embodiment, the apparatus for providing positive airway pressure therapy may further comprise (c) an arm pack adapted to be worn about the patient's arm, wherein the air pump system is mounted on the arm pack; (d) a rechargeable power supply unit wearable by the patient and adapted to supply power to drive the air pump system, wherein the rechargeable power supply unit is mounted on the arm pack; and (e) a hose adapted to carry air from the air pump system to the mask. Alternatively, instead of a rechargeable power supply unit, the apparatus may include an electrical supply line attached at one end to the air pump system for supplying electric power thereto and an electrical plug at the other end for insertion into an external power outlet.

In a related embodiment, the apparatus for providing positive airway pressure therapy may further comprise (c) a data monitoring system adapted to monitor operating data when the apparatus is in use by the patient; (d) an electrical supply line attached at one end to the air pump system for supplying electric power thereto and an electrical plug at the other end for insertion into an external power outlet; and (e) a data transmission wire adapted to transmit operating data from the data monitoring system to a base unit or other data receiving means, wherein the data transmission wire is generally parallel with the electrical line and optionally is enclosed together with the electrical line in a common sheath.

In another related embodiment, the apparatus for providing positive airway pressure therapy may further comprise connector means adapted for coupling the mask and the air pump system and for decoupling the mask from the air pump system such that the air pump system provides air to the mask when coupled with the mask.

In the above embodiments, the air pump system may comprise 4 to 100 individual elements, may comprise 100 to 1,000 individual elements, or may comprise greater than 1,000 individual elements.

A different embodiment of the invention relates to an apparatus for providing positive airway pressure comprising (a) a mask adapted for the delivery of pressurized air to maintain positive air pressure in a patient's airway; (b) an air pump system comprising a plurality of pump elements and adapted to supply pressurized air to the mask; and(c) a power supply unit adapted to drive the pump elements. The mask, air pump system, and power supply unit are combined in an integrated unit adapted to be worn on the patient's head.

In this embodiment, the apparatus may further comprise a base unit adapted to couple with the integrated unit when the integrated unit is not in use by the patient, wherein the base unit includes a recharging system adapted to recharge the power supply unit. The integrated unit may include a data monitoring and storage system to monitor and store operating data during use of the integrated unit by the patient. The base unit may include a data logging system adapted to download and store the data from the integrated unit while the base unit is coupled with the integrated unit. The integrated unit may include a data monitoring and wireless transmission system adapted to monitor and transmit operating data during use of the integrated unit by the patient and wherein the base unit includes a receiver adapted to receive and store the data transmitted by the wireless transmission system of the integrated unit.

In this embodiment, the apparatus may further comprise any of (1) a humidification system adapted to humidify inlet air to the air pump system or the pressurized air supplied to the mask; (2) a heating system adapted to heat inlet air to the air pump system or the pressurized air supplied to the mask; and (3) a filtration system adapted to filter inlet air to the air pump system or the pressurized air supplied to the mask.

Another embodiment of the invention includes an apparatus for providing positive airway pressure comprising (a) a mask for the delivery of pressurized air to maintain positive air pressure in a patient's airway; (b) an air pump system comprising a plurality of pump elements and adapted to supply pressurized air to the mask; and (c) a power supply unit connected to the air pump system by an electrical power supply line. The air pump system may be mounted on one of the mask, an arm pack adapted to be worn on the patient's arm, a cap or headpiece adapted to be worn on the patient's head, a vest adapted to be worn on the patient's torso, and a waist pack adapted to be worn around the patient's waist; the power supply unit may be mounted on one of the arm pack, the cap or headpiece, the vest, and the waist pack.

An optional embodiment of the invention relates to an apparatus for the supply of pressurized air to a mask comprising (a) an air pump system including a plurality of pump elements adapted to supply pressurized air to the mask; and (b) means for coupling the air pump system to the mask and for decoupling the integrated air pump system from the mask. In this embodiment, the air pump system may comprise 4 to 100 individual pump elements, or 100 to 1,000 individual pump elements, or greater than 1,000 individual pump elements. The apparatus may further comprise a power supply system adapted to provide power to drive the pump elements.

Another optional embodiment relates to a docking station for a positive airway pressure therapy device consisting essentially of (a) means for coupling and uncoupling the docking station and the device; (b) means for setting operating parameters for the device and means for transferring the operating parameters to the device; (c) means for downloading operating data from the device to the docking station; and (d) means for connecting the docking station to a source of electric power. The means for coupling and uncoupling the docking station and the device may include means for connecting and disconnecting an electrical power connector between the docking station and the device. The docking station may further comprise recharging means adapted to recharge a rechargeable power supply in the device when the device is coupled with the docking station.

A final embodiment of the invention relates to a method for providing positive airway pressure comprising
(a) providing an apparatus including (1) a mask for the delivery of pressurized air to maintain positive airway pressure in a patient's airway, and (2) an air pump system comprising a plurality of pump elements adapted to supply pressurized air to the mask, wherein the air pump system is attached directly to the mask or is adapted to be worn on a part of the patient's body spaced apart from the mask;
(b) mounting the mask on the patient's face; and
(c) compressing atmospheric air in the air pump system to provide pressurized air, introducing the pressurized air into the mask, and maintaining the positive airway pressure in the patient's airway during both inhalation and exhalation.

The pressure of the pressurized air in the mask during inhalation and exhalation may be controlled at a pressure between 4 and 25 cm water. Alternatively, the pressure of the pressurized air in the mask may be controlled at a first pressure during inhalation and a second pressure during exhalation, wherein the second pressure is less than the first pressure. The first and second pressures may be between 4 and 25 cm water.

The method may further comprise providing power to operate the air pump system by an electrical supply line from an external source. Alternatively, the method may further comprise providing power to operate the air pump system by an electrical supply line from a rechargeable power source worn on the patient's body.

### BRIEF DESCRIPTION OF SEVERAL VIEWS OF THE DRAWINGS

Embodiments of the invention are illustrated by the following drawings, which are not necessarily to scale.

Fig. 1 illustrates an embodiment of the invention showing an integrated mask, air pump system, and power supply system to supply pressurized air to a patient for positive airway pressure therapy.

Fig. 2 illustrates another embodiment of the invention showing a mask supplied with pressurized air from an air pump system and power supply system worn on a patient's head.

Fig. 3 illustrates an alternative embodiment of the invention showing a mask supplied with pressurized air from an air pump system and power supply system worn on a patient's torso.

Fig. 4 illustrates another alternative embodiment of the invention showing a mask supplied with pressurized air from an air pump system and power supply system worn about a patient's waist.

Fig. 5 illustrates a related embodiment of the invention showing a mask supplied with pressurized air from an air pump system and power supply system worn on a patient's upper arm.

Fig. 6 illustrates another related embodiment of the invention showing an integrated mask and air pump system wherein power for the air pump system is supplied from an external source.

Fig. 7 illustrates an embodiment of the invention wherein a rechargeable power supply is located on a patient's arm and wherein the air pump system is located on the mask.

Fig. 8A illustrates an exemplary individual diaphragm pump element that may be used in embodiments of the invention.

Fig. 8B illustrates an exemplary parallel configuration for a plurality of individual diaphragm pump elements that may be used in embodiments of the present invention.

### DETAILED DESCRIPTION OF THE INVENTION

The embodiments of the present invention provide improved comfort and ease of use of positive airway pressure devices by utilizing a compact, lightweight air pump system that utilizes a plurality of small individual air pump elements. This compact air pump system may be combined with a rechargeable power supply and the combined air pump-power supply system may be worn by the user to eliminate or reduce the length of the air hose, thereby providing a more comfortable user experience. In one embodiment, the compact air pump system may be directly combined with the mask and a lightweight rechargeable power supply to form an integrated unit that can be worn directly on the user's face and head. In this embodiment, the patient is not tethered to a base unit and there is no air hose. Alternatively, the air pump system and mask may be integrated so that the mask can be worn by the patient in the same manner as a conventional mask is worn, and power may be supplied by a thin electrical wire to the air pump system from a base unit or an electrical wall socket. In this embodiment, the thin electrical wire replaces the conventional air hose for increased patient comfort. In the first of these two alternatives, the compact air pump system and rechargeable power supply may be combined into a unit that can be attached to and detached from any conventional mask. In the second of these alternatives, the compact air pump system may be attached to and detached from any conventional mask.

In another embodiment, the air pump system may be combined with a rechargeable power supply to form an integrated air pump system that can be worn on the patient's body at a location spaced apart from the mask. The integrated air pump system may be installed, for example, in a vest designed to be worn about the patient's torso or in a belt assembly designed to be worn around the patient's waist. Alternatively, the integrated air pump system may be installed in a cap or headpiece designed to be worn on the patient's head. In another embodiment, the integrated air pump system may be designed to strap on the patient's upper arm. In each of these embodiments, a short hose from the integrated air pump system to the mask is used to provide pressurized air to the patient's nose and/or mouth, and a long air hose connecting the patient to a base unit is not required. When using the integrated air pump system of this embodiment, the patient may move about more freely during sleep and may walk to the bathroom without disconnecting the system from a base unit.

In an alternative embodiment, the air pump system may be designed to be worn by the patient at a location on the patient's body spaced apart from the mask. The air pump system may be installed, for example, in a vest designed to be worn about the patient's torso or in a belt assembly designed to be worn around the patient's waist. Alternatively, the air pump system may be installed in a cap or headpiece designed to be worn on the patient's head. In another embodiment, the air pump system may be designed to strap on the patient's arm. In each of these embodiments, a short hose from the air pump system to the mask is used to provide pressurized air to the patient's nose and/or mouth. Power may be supplied a thin electrical wire to the air pump system from a base unit or an electrical socket, and the patient is not tethered to a base unit by long air hose.

In the present disclosure, the term "airway" has the usual anatomical meaning of any passage which conducts air from the atmosphere to the patient's lungs. The term "positive airway pressure" means that the pressure at any location in the patient's airway at a point in the breathing cycle when the patient is using an embodiment of the present invention is greater than the pressure in the patient's airway at that location and that point in the breathing cycle when the patient is not using the embodiment of the present invention. The term "pressurized air" means air at a pressure above the atmospheric pressure surrounding the patient.

In the present disclosure, the term "mask" means a full face mask, a nasal mask, nasal prongs, a mouth mask, an oral mouthpiece, or any other noninvasive interface device designed and used to provide pressurized gas to the patient's airway. The peripheral edges of the mask form a seal or seals against the appropriate parts of the patient's face, mouth, and/or nose to maintain a superatmospheric pressure within the mask. Some air leakage may occur through the seal or seals in normal operation. As used herein, the face includes the nose and mouth, and the head includes any part of the patient above the neck. The terms "wearable", "worn on the patient's body", and "worn by the patient" mean that the devices described herein are attached directly to the patient, for example, as a mask attached with straps, an arm pack secured about the patient's arm, or a waist pack secured around the patient's waist. The terms also mean that the devices may be mounted on, i.e., attached to or inserted into, an article worn by the patient. Such an article may be, for example, a cap or headpiece worn on the patient's head or a vest worn on the patient's torso.

The terms "power supply", "power supply unit", and "power supply system" used herein are equivalent and may utilize batteries or fuel cells to generate electrical power to drive the air pump system. The air pump system may be driven by either AC or DC, and the electrical power may be supplied to the air pump system as either AC or DC.

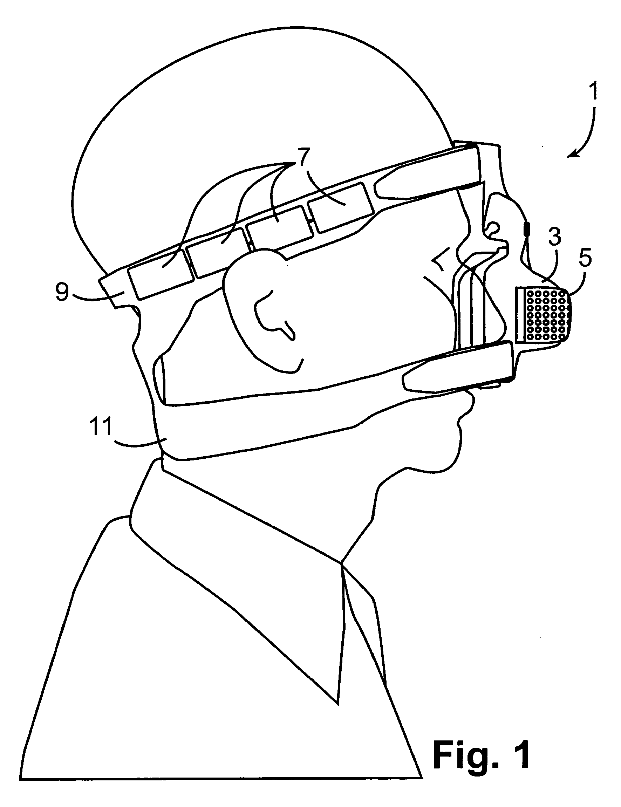

A first embodiment of the invention is illustrated schematically by the exemplary system of Fig. 1. Integrated positive airway pressure unit 1 comprises nasal mask 3 and air pump system 5; components of rechargeable power supply 7, which provide power to drive air pump system 5, are located on strap 9. Nasal mask 3 utilizes adjustable straps 9 and 11 to hold the mask against the patient's face and nose for proper sealing. In this example, the mask covers the nose or uses nasal prongs to pressurize the patient's airway through the nose. Alternatively, a full facial mask, a mouth mask, or an oral mouthpiece may be used instead of a nasal mask.

Air pump system 5, as well as the air pump systems in other embodiments of the invention, comprise a plurality of pump elements as described in more detail below. The air pump system also may include the necessary structure to the hold pump elements together in a stable assembly, air inlets and outlets for each pump element, power supply wiring to each pump element, a housing surrounding the pump elements, an optional on/off switch, and one or more manifolds or plenums to connect the outlets of the pump elements to a common pressurized air outlet.

Integrated positive airway pressure unit 1 may include an exhalation vent or port (not shown) of any type known in the art for the purpose of venting exhaled gas from the patient while maintaining an elevated pressure in the mask at an appropriate pressure for proper therapy. Alternatively, the design of air pump system 5 may include a period during the pump operating cycle for a patient's exhalation at the appropriate pressure.

The schematic drawing in Fig. 1 is not necessarily to scale and various alternative configurations of air pump system 5 and rechargeable power supply 7 are possible. For example, the individual pump elements of air pump system 5 may be arranged in a thin layer located over the mask and extending over the sides of the patient's face. This configuration would balance the mask components and distribute the component weight over a larger area of the patient's face and head for improved comfort.

In the embodiment of Fig. 1, the combined mask and air pump system of integrated positive airway pressure unit 1 is adapted to be worn facially by the patient, which means that the pump is supported by mask 3 that is in sealable contact with portions of the patient's nose and face around the nose. In this embodiment, air pump system 5 is mounted on or attached directly to mask 3 and components of rechargeable power supply 7 are located on strap 9. Alternatively, individual multiple elements of rechargeable power supply 7 may be arranged on or along the side portions of strap 11 or on both of the adjustable straps 9 and 11. The term "attached directly to" means that the air pump system is connected to and supported by mask 3 in a single combined assembly as illustrated in Fig. 1.

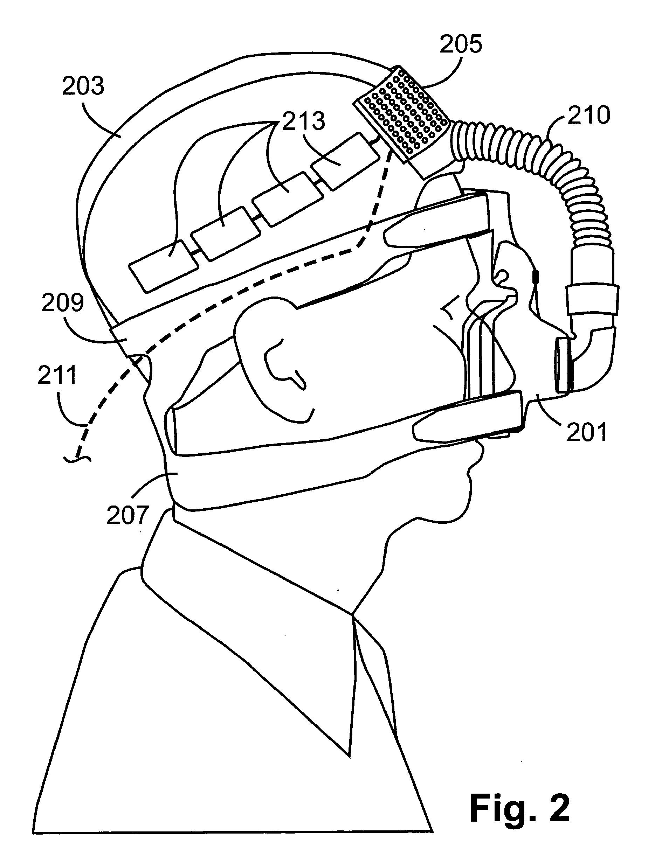

In a related alternative embodiment illustrated in Fig. 2, the integrated air pump system and power supply may be designed for placement in a hat or cap configuration as shown wherein nasal mask 201 is directly attached to the pump/power supply located on the hat or cap configuration of headpiece 203. Air pump system 205 is mounted on the front of headpiece 203 as shown and adjustable straps 207 and 209 may be used to secure mask 201 to headpiece 203 and to the patient's face. Air hose 210 transfers pressurized gas from air pump system 205 to mask 201. Components of rechargeable power supply 213 are mounted on headpiece 203 as shown. An exhalation vent or port (not shown) of any type known in the art may be included as part of mask 201 or as part of air pump system 205 in headpiece 203 for the purpose of venting exhaled gas from the patient while maintaining an elevated pressure in the mask at the required pressure for proper therapy. Alternatively, the design of air pump system 205 may include a period during the pump operating cycle for a patient's exhalation at an appropriate pressure.

In the embodiments of Figs. 1 and 2, the term "integrated" means that the air pump system and the power supply are combined and connected together in a single apparatus or assembly that can be worn on the patient's head.

Design variations of the exemplary pressure therapy systems characterized by Figs. 1 and 2 are possible and may be tailored to specific patient needs. For example, the embodiment of Fig. 2 may be modified by providing power to operate air pump system 205 by thin electrical supply wire 211 from a base unit, a conventional wall socket, or any other external power supply, i.e., a power supply separate from any systems worn by the patient. In this modification, rechargeable power supply 213 is not required.

Embodiments described in Figs. 1 and 2 are characterized by the feature that the mask, air pump system, and power supply are integrated to be worn on the patient's face or head as a single unit. Separate hose or power connections to a base unit or other external source are not required and the unit is totally self-contained. These designs are made possible by the use of compact air pump system 5 or 205 comprising a plurality of small, thin pump elements that can be arranged to fit the spatial configurations of Figs. 1 and 2 or modifications thereof. Multiple thin power supply elements may be combined in flexible power supply modules for rechargeable power supply 7 or 213 to fit the design requirements of the embodiments of Figs. 1 and 2. These air pump system and power supply elements are described in more detail below.

A variation of the system of Fig. 1 comprises connector means adapted for coupling the mask and the air pump system and for decoupling the mask from the air pump system such that the air pump system provides air to the mask when coupled with the mask. In this variation, it is possible for air pump system 5 to be connected with and disconnected from any commercially-available mask. This commercially-available mask can be used to provide the dual function of mask 3, i.e., (a) to supply pressurized air to the patient and (b) to provide a connection and support for the detachable air pump system 5. A variation of the system of Fig. 2 is possible wherein hose 210 is detachably connected to mask 201 such that mask 201 may be any commercially-available mask.

In alternative embodiments of the invention, the air pump system may be combined with a rechargeable power supply to form an integrated air pump system that can be worn on the patient's body at a location spaced apart from the mask. In these embodiments, the air pump system may designed and adapted to be worn, for example, in a vest about the patient's torso, in a belt assembly around the patient's waist, in a cap or headpiece on the patient's head, or in a module strapped on the patient's arm. In any of these embodiments, pressurized air is transferred from the integrated air pump system to the mask by a short, detachable, flexible hose of the proper diameter, for example, up to 2.5 cm in diameter. Any commercially-available mask may be used in these embodiments, which allows the patient to choose from a large number of available masks and to change mask type if necessary.

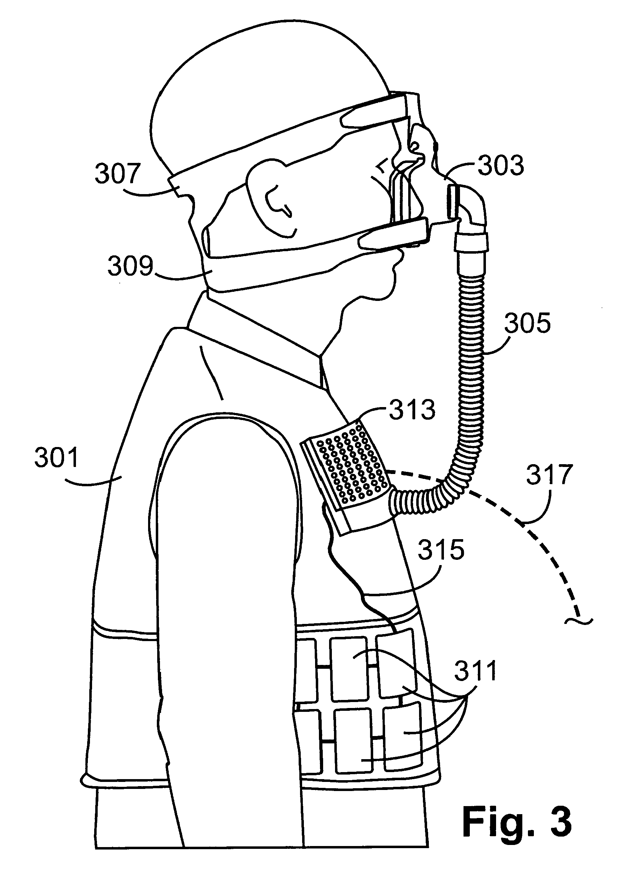

One of these alternative embodiments is illustrated in Fig. 3. Air pump system 313 and rechargeable power supply system 311 are installed on or within vest 301 and worn on the patient's torso as shown. Pressurized air is supplied to mask 303 via short, detachable, flexible hose 305. Power to operate air pump system 313 is provided via wire 315 from rechargeable power supply system 311. Mask 303 may be held in place on the patient's face by adjustable straps 307 and 309. An exhalation vent or port (not shown) of any type known in the art may be included as part of mask 303 or as part of air pump system 313 for the purpose of venting exhaled gas from the patient while maintaining an elevated pressure in the mask at the required pressure for proper therapy. Alternatively, the design of air pump system 313 may include a period during the pump operating cycle for a patient's exhalation at an appropriate pressure.

The vest in this embodiment may be a garment-type vest as illustrated in Fig. 3 or may be a section of material held to the patient's chest by straps around the shoulders and/or back. The meaning of the term "vest" is any device or assembly worn about the patient's torso wherein at least a portion of the device or assembly is located above the patient's waist.

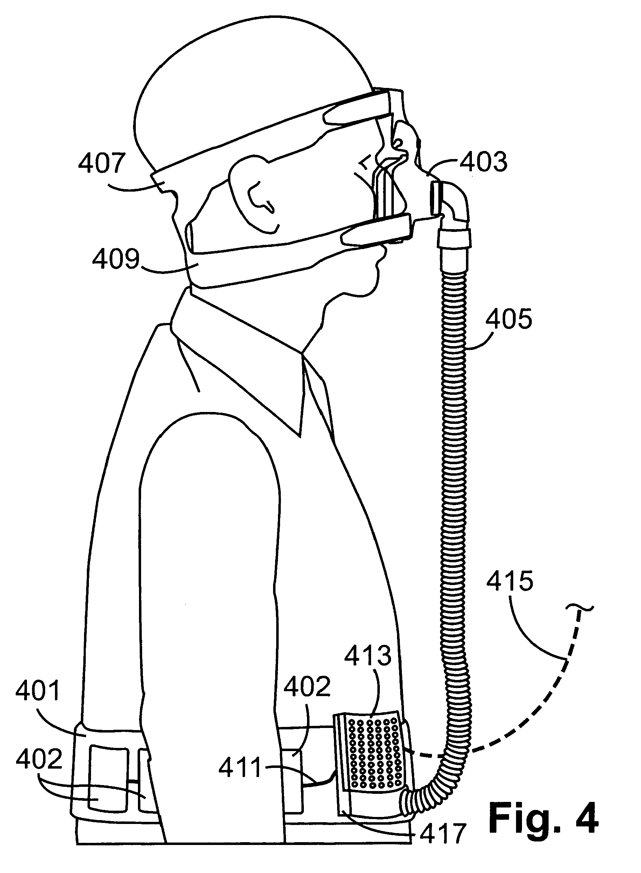

Another alternative embodiment is illustrated in Fig. 4. In this embodiment, air pump system 413 and rechargeable power supply system 402 are installed in waist pack 401 worn about the patient's waist as shown. Pressurized air is supplied to mask 403 via detachable, flexible hose 405. Mask 403 may be held in place on the patient's face by adjustable straps 407 and 409. Power to operate air pump system 413 may be provided via wire 411 from rechargeable power supply system 402. An exhalation vent or port (not shown) of any type known in the art may be included as part of mask 403 or as part of the air pump system 413 for the purpose of venting exhaled gas from the patient while maintaining an elevated pressure in the mask at the required pressure for proper therapy. Alternatively, the design of air pump system 413 in waist pack 401 may include a period during the pump operating cycle for a patient's exhalation at an appropriate pressure.

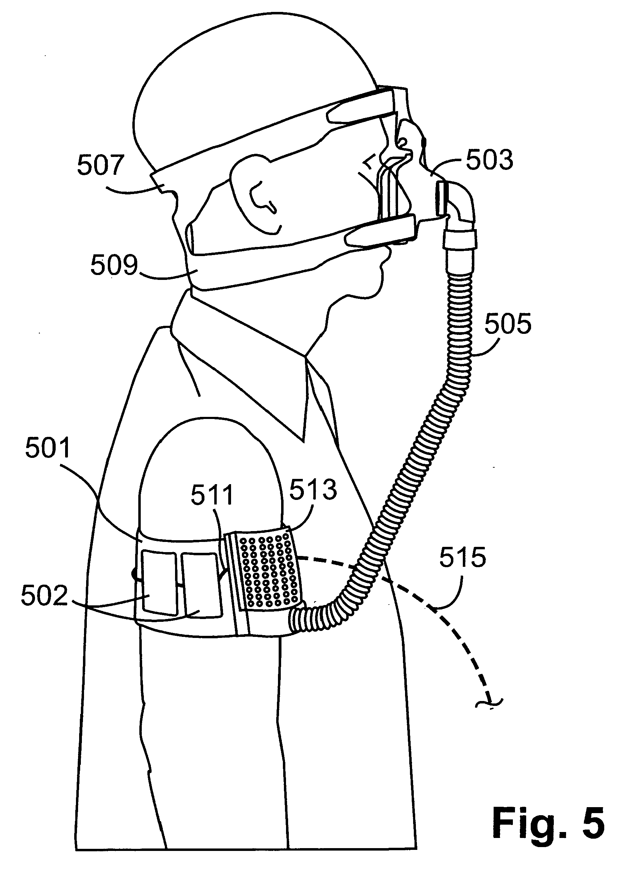

A related embodiment is illustrated in Fig. 5 utilizes arm pack 501 designed to be worm on the patient's upper arm. This pack contains air pump system 513 and rechargeable power supply system 502 designed and adapted to be installed in arm pack 501, which is worn about the patient's upper arm as shown. Pressurized air is supplied to mask 503 via detachable, flexible hose 505. Mask 503 may be held in place on the patient's face by adjustable straps 507 and 509. Power to operate air pump system 513 is provided via wire 511 from power supply system 502. As in the above embodiments, an exhalation vent or port (not shown) of any type known in the art may be included as part of mask 503 or as part of air pump system 513 for the purpose of venting exhaled gas from the patient while maintaining an elevated pressure in the mask at the required pressure for proper therapy. Alternatively, the design of air pump system 513 in arm pack 501 may include a period during the pump operating cycle for a patient's exhalation at an appropriate pressure.

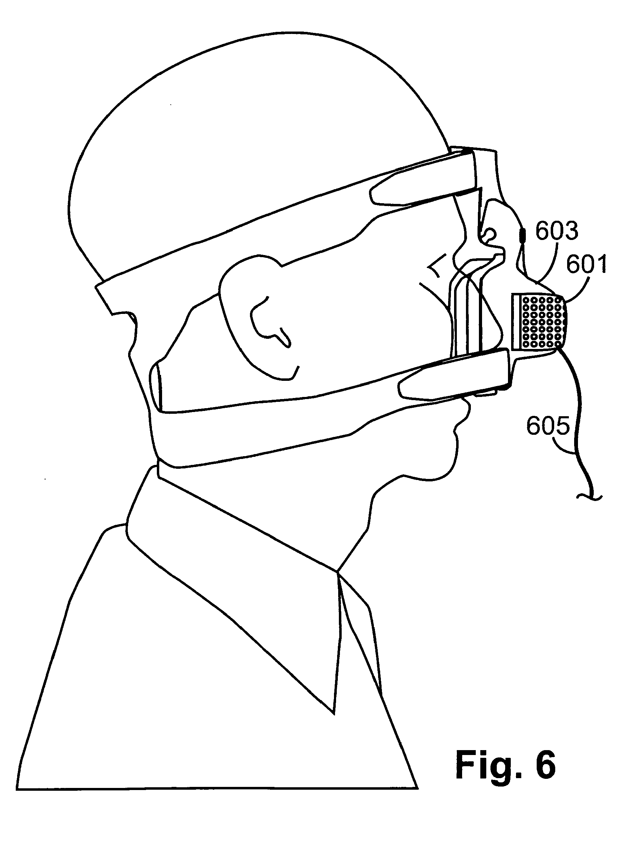

Alternatives to the embodiments illustrated above are possible wherein power to drive the air pump system is provided by a thin electrical line to the pump and a wearable power supply system is not used. For example, Fig. 6 illustrates one of these alternatives wherein air pump system 601 is mounted on mask 603 worn by the patient. Power to the air pump system may be provided via thin .electrical supply wire 605 from a base unit, a conventional wall socket, or any other external power supply. In any of these alternatives, the electrical supply wire may have an electrical plug suitable for insertion into an AC power outlet such as a typical wall outlet. Alternatively, the electrical plug may be suitable for insertion into a DC power outlet such as a 12V power outlet in a motor vehicle. If the air pump system operates on DC and the power outlet is AC, the electrical plug may include an AC/DC converter and/or may include a voltage regulator: Alternatively, but less desirably, the air pump system may include an AC/DC converter and/or may include a voltage regulator.

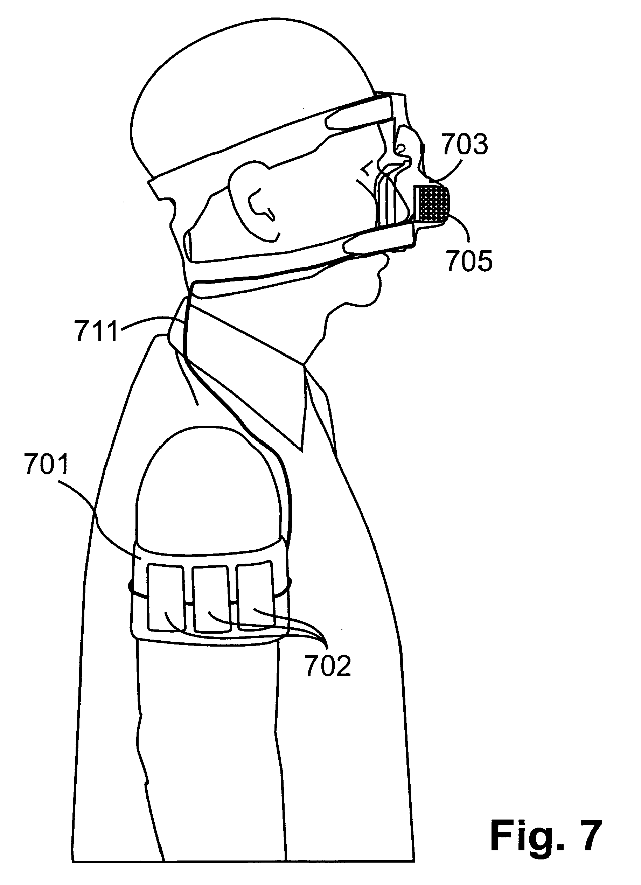

If desired in the embodiment of Fig. 6, a rechargeable power supply may be used to supply power via wire 605; this rechargeable power supply may be worn by the patient in a cap arrangement similar to that of Fig. 2, a vest arrangement similar to that of Fig. 3, a waist pack similar to that of Fig. 4, or an arm pack similar to that of Fig. 5. This latter embodiment is illustrated in Fig. 7, wherein arm pack 701 contains rechargeable power supply 702 that provides power via wire 711 to air pump system 705 mounted on mask 703.

In related embodiments, the system of Fig. 2 may be modified so that cap 203 is designed to contain only an air pump system; rechargeable power supply 213 is not used. Instead, power to operate air pump system 205 is provided by thin electrical supply wire 211 connected to a base unit, a conventional wall socket, or any other external power supply. The system of Fig. 3 may be modified so that vest 301 is designed to contain only an air pump system; rechargeable power supply 311 is not used. Instead, power to operate air pump system 313 is provided by thin electrical supply wire 317 connected to a base unit, a conventional wall socket, or any other external power supply. The embodiment of Fig. 4 may be modified in a similar manner wherein waist pack 401 is designed to contain only air pump system 413; rechargeable power supply 402 is not used. Instead, power to operate the air pump system is provided by thin electrical supply wire 415 connected to a base unit, a conventional wall socket, or any other external power supply. Similarly, the embodiment of Fig. 5 may be modified in a similar manner wherein arm pack 501 is designed to contain only air pump system 513; rechargeable power supply 502 is not used. Instead, power to operate the air pump system is provided by thin electrical supply wire 515 connected to a base unit, a conventional wall socket, or any other external power supply.

While the embodiments described above utilize specific locations of the air pump system and power supply system mounted on the cap, vest, arm pack, or waist pack worn by the patient, any other combination for the locations of the air supply system and power supply system is possible. In one example, the air pump system may be mounted on the arm pack and the power supply system may be mounted on the vest. In another example, the air pump system may be mounted on the mask and the power supply system may be mounted on the vest. In the most general description of all possible combinations, the air pump system may be mounted on the mask, the arm pack adapted to be worn on the patient's arm, the cap or headpiece adapted to be worn on the patient's head, the vest adapted to be worn on the patient's torso, or the waist pack adapted to be worn around the patient's waist; the power supply unit may be mounted on the arm pack, the cap or headpiece, the vest, or the waist pack.

Optionally, any of the embodiments described above that provide power to the air pump system via an electrical line from an external source may include a data transmission wire adapted to transmit operating data from the pump system to a base unit or other data receiving means, wherein the data transmission wire may be generally parallel with, i.e., following approximately same path as, the electrical line and may be enclosed together with the electrical line in a common sheath.

Pressurized air may be supplied to the mask in the embodiments described above by the air pump system in a typical pressure range of 4 to 25 cm water. Appropriate pressure control means known in the art may be used to provide constant pressure, dual pressure, or variable pressure therapy as required. The flow rate of pressurized air delivered to the mask will depend on the air leakage rate around the edges of the mask. Typically, the air pump system may be designed to supply 10 to 120 liter/min (specified at 23°C and atmospheric pressure) to the mask.

The embodiments of the invention described above are characterized by a common feature wherein the air pump system is adapted to be worn by the patient at various body locations. This feature eliminates the need for a long air hose from the mask to a base unit at the patient's bedside. In some embodiments, a power supply system also may be worn by the patient to supply power to the air pump system.

A compact, wearable air pump system for these embodiments may be designed using a plurality of small pump elements in an array that can be interconnected in series and/or parallel flow operation to provide the required air pressure and flow rates. The plurality of pump elements may be combined in relatively thin arrays or configurations that can be shaped to fit the various locations on the patient's body as described above. The individual pump elements may be any type of small or miniaturized pump selected from, but not limited to, piezoelectric pumps, thermopneumatic pumps, ultrasonically-driven pumps, electrostatic pumps, electro-osmosis pumps, electrohydrodynamic pumps, electromagnetic pumps, rotary pumps, shape memory alloy pumps, bimetallic pumps, diaphragm pumps, rotary vane pumps, scroll pumps, solenoid pumps, stepper-motor actuated pumps, piston pumps, and linear pumps.

Arrays of air pump elements suitable for use with embodiments of the present invention include a plurality of individual pump elements, i.e., include two or more elements. An array may comprise at least 4 individual pump elements and may include up to 20, up to 100, up to 1,000, or even more than 1,000 individual pump elements. For example, the plurality of pump elements may comprise 4 to 100 individual elements, 100 to 1,000 Individual elements, or greater than 1,000 individual elements. The individual pump elements should be capable of starting or stopping the flow of pressurized gas very quickly, for example, in 2 seconds or less, 0.5 second or less, or even 0.1 second or less.

The individual pump elements in a given array may be arranged in a parallel configuration wherein each pump element has the same or nearly the same inlet and discharge pressure, and the elements may have common inlet and/or outlet plenums or manifolds in certain design configurations. The number of parallel elements may be selected to produce any desired pressurized gas flow rate. Alternatively, individual pump elements may be arranged in a series configuration wherein the discharge of a given element feeds the inlet of the next element and the number of series elements may be selected to produce any desired gas pressure. Advantageously, the pump elements may be arranged in a matrix array comprising both parallel and series elements such that any combination of product flow rate and product pressure can be attained. The number of operating elements in the array may be varied with time to meet changing product flow and pressure requirements. Higher flow rates may require a greater number of operating parallel elements and higher pressures may require a greater number of operating series elements; conversely, lower flow rates may require a smaller number of operating parallel elements and lower pressures may require a smaller number of operating series elements. The numbers of both series and parallel elements in operation may be varied simultaneously when both flow and pressure requirements change with time to provide efficient turn-down and minimize power consumption.

In embodiments of the invention described above with reference to Figs. 1-7, for example, the individual pump elements for the air pump systems may be small diaphragm pumps adapted for parallel operation, series operation, or both parallel and series operation. The pump elements may be electrostatically activated or piezoelectrically activated. Small diaphragm pump elements that may be used in these embodiments, for example, are described in a paper entitled "The Dual Diaphragm Pump" by C. Cabuz et al, Technical Digest MEMS 2001, 14^{th} IEEE International Conference on Micro Electro Mechanical Systems, 21-25 January 2001, Interlaken, Switzerland, pp. 519-522, which is incorporated herein by reference. Further descriptions of small diaphragm-type pump elements are given in U.S. Patent Nos. 6,106,245, 6,179,586, 6,729,856, and 6,767,190, all of which are incorporated herein by reference. An alternative exemplary diaphragm pump element suitable for use in these embodiments is the Model MPD 1304 piezo-activated micropump marketed by thinXXS GmbH of Mainz, Germany.

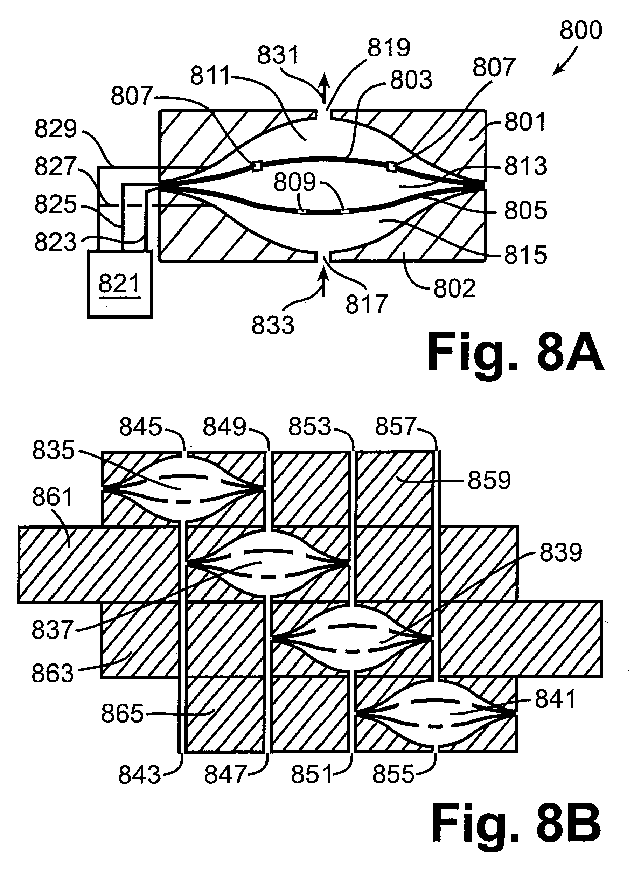

A non-limiting example of a multiple diaphragm-type pump element that may be used with embodiments of the present invention is illustrated in Fig. 8A, which is adapted from Figure 1 of the paper by C. Cabuz et al. identified above. Fig. 8A is a cross-sectional side view of pump element 800 which comprises upper body section 801 and lower body section 802 wherein the body sections define a lens-shaped inner cavity. Upper membrane 803 and lower membrane 805 are fixed or clamped at their peripheries between upper body section 801 and lower body section 802. The inner cavity and the membranes are circular when viewed from the top. Upper membrane 803 has one or more holes or openings 807 and lower membrane has one or more holes or openings 809. No opening in the upper membrane is congruent or coincident with an opening in the lower membrane; when the membranes are in contact, the openings in one membrane are covered by the other membrane. Each membrane and body section has electrodes which allow each membrane to be moved electrostatically and independently in both vertical directions by imposing appropriate voltage potentials between the membranes and the upper and lower body sections.

The voltage potentials for driving the membranes are provided by voltage control unit 821 via electrical leads 823 to lower membrane 805, 825 to upper membrane 803, 827 to lower body section 802, and 829 to upper body section 801. By appropriate control of these voltage potentials by means of voltage control unit 821, the membranes can be moved independently. Exemplary dimensions of pump element 800 may include a width of 0.5 to 3 cm and a height of 0.05 to 0.2 cm; the pump element may provide 10 to 50 std cc/min of compressed gas at pressures up to about 25 cm water. Higher pressures may be generated as required.

Pump element 800 may be operated in an exemplary gas compression cycle wherein air or any other gas is drawn through inlet 817 into the lens-shaped cavity and discharged as compressed gas through outlet 819. One exemplary cycle comprises intake and compression steps defined by the phases of membrane positions and movement as follows.
(1) In a first phase, the voltage potentials between the membranes and lower body section 802 are set so that the bottom surface of lower membrane 805 contacts the inner surface of lower body section 802 and the lower surface of upper membrane 803 contacts the upper surface of lower membrane 805, thereby closing holes 807 of the upper membrane and holes 809 of the lower membrane.
(2) The voltage potentials are changed so that the upper and lower membranes remain in contact and move together in an upward direction, thereby compressing gas initially present in the cavity and discharging the resulting compressed gas 831 through outlet 819. As the two membranes move upward in tandem, intake gas 833 is drawn via inlet 817 into the cavity below the membranes. This combined intake-discharge stroke is completed as the two membranes reach the inner surface of upper body section 801.
(3) The voltage potentials are changed so that upper membrane 803 remains in contact with the inner surface of upper body section 801 and lower membrane 805 moves downward and contacts the inner surface of lower body section 802. Gas below the lower membrane flows through holes 809 as the membrane moves downward.
(4) The voltage potentials are changed so that upper membrane 803 moves downward. Gas below the upper membrane flows through holes 807 as the upper membrane moves downward. At the completion of this step, the membranes return to the positions described in phase (1) above wherein the bottom surface of lower membrane 805 contacts the inner surface of lower body section 802 and the lower surface of upper membrane 803 contacts the upper surface of lower membrane 805, thereby closing holes 807 of the upper membrane and holes 809 of the lower membrane.

Phases (1) through (4) are repeated in a cyclic manner at a high frequency, for example, between about 30 and 100 cycles per second. While this exemplary compression cycle utilizes vertical movement of the membranes and is described in terms of upper and lower elements relative to the horizontal, the pump element and membranes may have any orientation relative to the horizontal and may be operated in any orientation relative to the horizontal.

A plurality of the individual pump elements of Fig. 8A may be arranged in a parallel configuration wherein each pump element has the same or nearly the same inlet and discharge pressure, and the elements may have common inlet and/or outlet plenums or manifolds in certain design configurations. The number of parallel elements may be selected to produce any desired pressurized gas flow rate. Alternatively, the individual pump elements may be arranged in a series configuration wherein the discharge of a given element feeds the inlet of the next element and the number of series elements may be selected to produce any desired gas pressure. Advantageously, the pump elements may be arranged in a matrix array comprising both parallel and series elements such that any combination of product flow rate and product pressure can be attained. The number of operating elements in the array may be varied with time to meet changing product flow and pressure requirements. Higher flow rates may require a greater number of operating parallel elements and higher pressures may require a greater number of operating series elements; conversely, lower flow rates may require a smaller number of operating parallel elements and lower pressures may require a smaller number of operating series elements. The numbers of both series and parallel elements in operation may be varied simultaneously when both flow and pressure requirements change with time.

A section of an exemplary parallel combination of multiple pump elements of Fig. 8A is illustrated in Fig. 8B. Four pump elements 835, 837, 839, and 841 are combined in overlapping fashion as shown to provide a pattern that can be repeated to give a large number multiple elements operating in parallel. Pump element 835 has inlet 843 and outlet 845, pump element 837 has inlet 847 and outlet 849, pump element 839 has inlet 851 and outlet 853, and pump element 841 has inlet 855 and 857. The overlapping parallel configuration of the pump elements is effected by spacers 859, 861, 863, and 865, wherein appropriate passages are formed in the spacers to connect with the pump element inlets and outlets as shown.

A plurality of parallel pump elements having the orientation illustrated in Fig. 8B, for example, may be installed in the air pump systems of Figs. 1-7. As an illustration, air pump system 413 of Fig. 4 shows a front intake grille having multiple small opening. A layer of multiple parallel pump elements as illustrated in Fig. 8B may be installed behind and generally parallel with the grille such that the intakes of all pump elements are in direct flow communication with the atmosphere surrounding the patient. The discharges of all pump elements may lead into rear plenum 417 that is located behind the parallel pump array. The rear plenum is adjacent to, and may be attached to, the surface of waist pack 401 worn by the patient. Plenum 417 extends to and is connected with the end of hose 405.

In the embodiments of Figs. 1-5 and 7, the power supply unit or system may utilize disposable or regenerable power supply elements such as batteries or fuel cells. Typically, the power supply is regenerable. Rechargeable batteries of any type may be used such as, for example, alkaline, nickel-cadmium, lithium ion, nickel metal hydride, and lithium polymer batteries. Battery life between full charge and discharge should be at least 5 hours and may be greater than 8 hours. Thin, flexible arrays of regenerable batteries may be designed and appropriately shaped to fit various parts of a patient's body as shown in Figs. 1-5 and 7. In Fig. 1, for example, a plurality of thin rechargeable power elements or batteries are shown as power supply 7.

Small fuel cell systems powered by hydrogen or methanol may be used to produce power to operate the air pump system. The fuel cell system would include a refillable fuel vessel and would typically hold enough fuel to operate the air pump system for at least 5 hours and optionally greater than 8 hours.

The embodiments of the present invention may be operated according to any pressure-time profile known in the art of positive airway pressure therapy. In one mode of operation, continuous positive airway pressure (CPAP) is maintained at a selected relatively constant pressure during the breathing cycle. In another mode, bi-level positive airway pressure air is supplied to the patient at two selected pressures ― a higher pressure during inhalation and a lower pressure during exhalation. In another operating mode, the air pressure may be varied during the breathing cycle to meet a patient's specific needs as described in U.S. Patent 6,530,372, which is incorporated herein by reference. For the current embodiments, the pressure drop measurement may be taken by a relative pressure transducer located either on the mask or near the discharge point of the air pump system. The reading from the relative pressure transducer would be compared against the relative pressure set point (which may be constant or varying, depending upon the selected operating mode). The air pump system would then be adjusted to achieve a flow rate which moved the relative pressure towards the set point. The adjustment may be a change in the speed of the individual pump elements, activation or deactivation of series or parallel sections of the array, or a combination of these two methods.

The embodiments of the present invention may include a data monitoring and storage system to monitor and store operating and compliance data during use of the positive airway pressure therapy device by the patient. This data monitoring and storage system may include the capability to track date and time, and may be integrated with the mask or air pump system to measure and store gas pressures and flow rates as a function of time during the patient's sleep period. As is common with conventional positive airway pressure devices, the data monitoring system can provide date and time stamp, mask-off events, individual apnea events, and pressure requirements.
Optionally, this data monitoring system may include a wireless transmission system adapted to monitor and transmit real-time operating data during use of the integrated unit by the patient.

An embodiment of the invention includes a system for the supply of pressurized air to a mask comprising (1) an air pump system that includes a plurality of pump elements adapted to supply pressurized air to the mask and (2) means for coupling the air pump system to the mask and for decoupling the integrated air pump system from the mask. This allows the pressurized air supply system to be used with any commercially-available mask. The apparatus may comprise 4 to 100 individual pump elements, or may comprise 100 to 1,000 individual elements, or may comprise greater than 1,000 individual elements. The apparatus may also include a power supply system adapted to provide power to drive the pump elements.

Another embodiment of the invention includes a docking station for the positive airway pressure therapy device consisting essentially of (a) means for coupling and uncoupling the docking station and the device; (b) means for setting operating parameters for the device and means for transferring the operating parameters to the device; (c) means for downloading operating data from the device to the docking station; and (d) means for connecting the docking station to a source of electric power. In contrast with prior art devices, this docking station does not include a blower, air pump, or any other air moving device.

The positive airway pressure therapy device may be adapted to couple with the docking station or base unit when the device is not in use by the patient. This docking station may include a data logging system adapted to download and store the operating data from the integrated unit while coupled with the docking station. Data storage capability of the docking station can hold 365 nights or more of monitoring results. The docking station may include a recharging system adapted to recharge the power supply unit when the air supply system is coupled with the docking station. The recharging system will be a battery charger when the air supply system is driven by a battery-operated pump system. Alternatively, when the air supply system is driven by power generated by a fuel cell system, the docking station may include a fuel storage tank and means for transferring fuel to the fuel vessel of the fuel cell system. When the data monitoring system associated with the mask or air pump system includes a wireless transmission system adapted to monitor and transmit real-time operating data, the docking station will include a receiver adapted to receive and store the data transmitted by the wireless transmission system of the mask or air pump system. The docking station may include a power outlet socket adapted to receive a plug on electrical supply wire 211, 317, 415, 515, or 605 in embodiments of Figs. 2, 3, 4, 5, and 6, respectively.

The docking station also may include means to set operating parameters for the integrated air supply unit and transmit these settings to the air supply system for storage when it is coupled with the docking station. These operating parameters may include, for example, the pressurized air flow rate, the air pressure if operating in the CPAP mode, the upper and lower air pressures if operating in the bi-level positive airway pressure mode, and other parameters as necessary.

A humidification system may be included in any of the embodiments of Figs. 1-7 to humidify the pressurized air supplied to the patient. This may be effected by a water evaporation system installed, for example, at the outlet of the air pump system so that pressurized humidified air can be supplied directly to the mask. Alternatively, in the embodiments of Figs. 3-5, the humidification system may be installed in the mask. If desired, the humidification system may be installed at the inlet to the air pump system. The humidification system should hold enough water for at least 5 hours and possibly greater than 8 hours of operation. Humidification may be effected by a sponge placed in the air flow path and fed by a water reservoir. Alternatively, a miniature atomizer may mist water into the air flow path. Alternatively, a small heater and bubbler system may be used.

Optionally, a heating system may be included to heat the air before or after pressurization. The heat may be provided by a simple resistive heating element placed in the air flow path or by other means known in the art. A thermocouple or other temperature measuring device may be placed after the heater to provide control feedback to regulate power to the heater.

Altitude compensation may be included in the operating control system so that the pressure of the air delivered to the patient can be changed in response to an increase or decrease in atmospheric pressure. In another option, an air filtration system may be included to filter the air before or after pressurization.

To minimize weight on the mask for embodiments represented in Figs. 1, 2, 6, and 7, the parts of the system for control and data logging worn on the mask may include only an on-off switch, with the remaining parts located on the mask straps or headpiece. A programming and data transfer interface may be provided by external means located, for example, on a docking station rather than on the mask or headgear. In the embodiments shown in Figs. 3, 4, or 5, the programming interface may be provided on vest 301, waist pack 401, or arm pack 501, respectively.

### EXAMPLE

The following Example illustrates embodiments of the present invention and does not limit these embodiments to any of the specific details described therein.

A pressurized air supply system utilizing the embodiment of Fig. 3 is designed to provide up to 100 Ipm of air at pressures between 2 and 20 cm water and to operate in the CPAP mode. The system supplies the pressurized air to a sleep apnea patient for 8 hours while the patient sleeps. Based on the performance specifications from the paper of C. Cabuz et al cited above, each individual pump element of air pump system 313 can deliver pressurized air at 30 ml/min, requires 8 mW of power, and has overall dimensions of 1.5 cm x 1.5 cm x 0.1 cm. Each pump element generates air at a pressure up to 20 cm water, and 3,333 pump elements are operated in parallel to provide the required pressurized air flow rate of 100 Ipm. The overall volume of air supply system 313 is 750 ml and the system is configured in a flat array 1 cm thick by 27.4 cm square. The system is mounted on the chest portion of vest 301 as shown in Fig. 3. The 3,333 pump elements are driven by power supply 311 using lithium ion rechargeable batteries to provide approximately 26.7 watts total. Voltage conversion and control circuitry consumes an additional 5 watts for a total power consumption of 31.7 watts.

The batteries in power supply 311 are Ultralife model UBP103450/PCM, each of which has a nominal capacity of 6.7 watt-hr at an average of 3.7 volts and weighs 40 grams. For 8 hours of operation at 31.7 watts, 37.9 batteries would be required in parallel. In practice, batteries are discrete units, so the configuration in this Example uses 7 parallel sets of 6 batteries in series for a total of 42 batteries having a total weight of 1.68 kilograms. The total volume of the batteries is 898 ml and the 42 batteries are arranged in a layer 0.11 cm thick mounted on the lower portion of vest 301.

## Claims

1. An apparatus for providing positive airway pressure therapy comprising
(a) a mask adapted for the delivery of pressurized air to maintain positive airway pressure in a patient's airway; and
(b) an air pump system comprising a plurality of pump elements adapted to supply pressurized air to the mask;
wherein the air pump system is attached directly to the mask or is adapted to be worn on a part of the patient's body spaced apart from the mask.

2. The apparatus of Claim 1 wherein the plurality of pump elements are selected from the group consisting of piezoelectric pumps, thermopneumatic pumps, ultrasonically-driven pumps, electrostatic pumps, electro-osmosis pumps, electrohydrodynamic pumps, electromagnetic pumps, rotary pumps, shape memory alloy pumps, bimetallic pumps, diaphragm pumps, rotary vane pumps, scroll pumps, solenoid pumps, stepper-motor actuated pumps, piston pumps, and linear pumps.

3. The apparatus of Claim 2 wherein the pump elements are diaphragm pumps adapted for parallel operation, series operation, or both parallel and series operation.

4. The apparatus of Claim 3 wherein the diaphragm pumps are electrostatically activated or piezoelectrically activated.

5. The apparatus of Claim 1 which further comprises a rechargeable power supply unit wearable by the patient and adapted to supply power to drive the air pump system,
wherein the rechargeable power supply unit comprises one or more batteries.

6. The apparatus of Claim 1 which further comprises a rechargeable power supply unit wearable by the patient and adapted to supply power to drive the air pump system,
wherein the rechargeable power supply unit comprises one or more fuel cells.

7. The apparatus of Claim 1 which further comprises
(c) a rechargeable power supply unit wearable by the patient and adapted to supply power to drive the air pump system; and
(d) a base unit adapted to couple with the apparatus for providing positive airway pressure when the apparatus is not in use by the patient, wherein the base unit includes a recharging system adapted to recharge the power supply unit.

8. The apparatus of Claim 7 wherein the apparatus for providing positive airway pressure includes a data monitoring and storage system to monitor and store operating data when the apparatus is in use by the patient.

9. The apparatus of Claim 8 wherein the base unit includes a data logging system adapted to download and store the data from the apparatus for providing positive airway pressure while the base unit is coupled with the apparatus for providing positive airway pressure.

10. The apparatus of Claim 7 wherein the apparatus for providing positive airway pressure further comprises a data monitoring and wireless transmission system adapted to monitor and transmit operating data while the apparatus is in use by the patient and
wherein the base unit includes a receiver adapted to receive and store the data transmitted by the wireless transmission system.

11. The apparatus of Claim 1 which further comprises any of
(1) a humidification system adapted to humidify inlet air to the air pump system or the pressurized air supplied to the mask;
(2) a heating system adapted to heat inlet air to the air pump system or the pressurized air supplied to the mask; and
(3) a filtration system adapted to filter inlet air to the air pump system or the pressurized air supplied to the mask.

12. The apparatus of Claim 1 wherein the air pump system is attached to and mounted on the mask to form an integrated mask and air pump system.

13. The apparatus of Claim 12 wherein the apparatus further comprises a rechargeable power supply unit wearable by the patient and adapted to supply power to drive the air pump system and an electric supply wire to provide power from the power supply unit to the air pump system.

14. The apparatus of Claim 13 wherein the integrated mask and air pump system comprises one or more straps adapted to secure the mask to the patient's face, and
wherein the rechargeable power supply is mounted on at least one of the one or more straps.

15. The apparatus of Claim 13 wherein the rechargeable power supply unit is mounted on an arm pack adapted to be worn on the patient's arm.

16. The apparatus of Claim 13 wherein the rechargeable power supply unit is mounted on a cap or headpiece adapted to be worn on the patient's head.

17. The apparatus of Claim 13 wherein the rechargeable power supply unit is mounted on a vest adapted to be worn on the patient's torso.

18. The apparatus of Claim 13 wherein the rechargeable power supply is mounted on a waist pack adapted to be worn around the patient's waist.

19. The apparatus of Claim 12 which further comprises an electrical supply line attached at one end to the air pump system for supplying electric power thereto and an electrical plug at the other end for insertion into an external power outlet.

20. The apparatus of Claim 1 which further comprises
(c) a cap or headpiece adapted to be worn on the patient's head, wherein the air pump system is mounted on the cap or headpiece;
(d) a rechargeable power supply unit wearable by the patient and adapted to supply power to drive the air pump system, wherein the rechargeable power supply unit is mounted on the cap or headpiece; and
(e) a hose adapted to carry air from the air pump system to the mask.

21. The apparatus of Claim 1 which further comprises
(c) a cap or headpiece adapted to be worn on the patient's head, wherein the air pump system is mounted on the cap or headpiece;
(d) a hose adapted to carry air from the air pump system to the mask; and
(e) an electrical suppy line attached at one end to the air pump system for supplying electric power thereto and an electrical plug at the other end for insertion into an external power outlet.

22. The apparatus of Claim 1 which further comprises
(c) a vest adapted to be worn about the patient's torso, wherein the air pump system is mounted on the vest;
(d) a rechargeable power supply unit wearable by the patient and adapted to supply power to drive the air pump system, wherein the rechargeable power supply unit is mounted on the vest; and
(e) a hose adapted to carry air from the air pump system to the mask.

23. The apparatus of Claim 1 which further comprises
(c) a vest adapted to be worn about the patient's torso, wherein the air pump system is mounted on the vest;
(d) a hose adapted to carry air from the air pump system to the mask; and
(e) an electrical supply line attached at one end to the air pump system for supplying electric power thereto and an electrical plug at the other end for insertion into an external power outlet.

24. The apparatus of Claim 1 which further comprises
(c) a waist pack adapted to be worn about the patient's waist, wherein the air pump system is mounted on the waist pack;
(d) a rechargeable power supply unit wearable by the patient and adapted to supply power to drive the air pump system, wherein the rechargeable power supply unit is mounted on the waist pack; and
(e) a hose adapted to carry air from the air pump system to the mask.

25. The apparatus of Claim 1 which further comprises
(c) a waist pack adapted to be worn about the patient's waist, wherein the air pump system is mounted on the waist pack;
(d) a hose adapted to carry air from the air pump system to the mask; and
(e) an electrical supply line attached at one end to the air pump system for supplying electric power thereto and an electrical plug at the other end for insertion into an external power outlet.

26. The apparatus of Claim 1 which further comprises
(c) an arm pack adapted to be worn about the patient's arm, wherein the air pump system is mounted on the arm pack;
(d) a rechargeable power supply unit wearable by the patient and adapted to supply power to drive the air pump system, wherein the rechargeable power supply unit is mounted on the arm pack; and
(e) a hose adapted to carry air from the air pump system to the mask.

27. The apparatus of Claim 1 which further comprises
(c) an arm pack adapted to be worn on the patient's arm, wherein the air pump system is mounted on the arm pack;
(d) a hose adapted to carry air from the air pump system to the mask; and
(e) an electrical supply line attached at one end to the air pump system for supplying electric power thereto and an electrical plug at the other end for insertion into an external power outlet.

28. The apparatus of Claim 1 which further comprises
(c) a data monitoring system adapted to monitor operating data when the apparatus is in use by the patient;
(d) an electrical supply line attached at one end to the air pump system for supplying electric power thereto and an electrical plug at the other end for insertion into an external power outlet; and
(e) a data transmission wire adapted to transmit operating data from the data monitoring system to a base unit or other data receiving means, wherein the data transmission wire is generally parallel with the electrical line and optionally is enclosed together with the electrical line in a common sheath.

29. The apparatus of Claim 1 which further comprises connector means adapted for coupling the mask and the air pump system and for decoupling the mask from the air pump system such that the air pump system provides air to the mask when coupled with the mask.

30. The apparatus of Claim 1 wherein the air pump system comprises 4 to 100 individual elements.

31. The apparatus of Claim 1 wherein the air pump system comprises 100 to 1,000 individual elements.

32. The apparatus of Claim 1 wherein the air pump system comprises greater than 1,000 individual elements.

33. An apparatus for providing positive airway pressure comprising
(a) a mask adapted for the delivery of pressurized air to maintain positive air pressure in a patient's airway;
(b) an air pump system comprising a plurality of pump elements and adapted to supply pressurized air to the mask; and
(c) a power supply unit adapted to drive the pump elements;
wherein the mask, air pump system, and power supply unit are combined in an integrated unit adapted to be worn on the patient's head.

34. The apparatus of Claim 33 which further comprises a base unit adapted to couple with the integrated unit when the integrated unit is not in use by the patient, wherein the base unit includes a recharging system adapted to recharge the power supply unit.

35. The apparatus of Claim 34 wherein the integrated unit includes a data monitoring and storage system to monitor and store operating data during use of the integrated unit by the patient.

36. The apparatus of Claim 35 wherein the base unit includes a data logging system adapted to download and store the data from the integrated unit while the base unit is coupled with the integrated unit.

37. The apparatus of Claim 34 wherein the integrated unit includes a data monitoring and wireless transmission system adapted to monitor and transmit operating data during use of the integrated unit by the patient and wherein the base unit includes a receiver adapted to receive and store the data transmitted by the wireless transmission system of the integrated unit.

38. The apparatus of Claim 33 which further comprises any of
(1) a humidification system adapted to humidify inlet air to the air pump system or the pressurized air supplied to the mask;
(2) a heating system adapted to heat inlet air to the air pump system or the pressurized air supplied to the mask; and
(3) a filtration system adapted to filter inlet air to the air pump system or the pressurized air supplied to the mask.

39. An apparatus for providing positive airway pressure comprising
(a) a mask for the delivery of pressurized air to maintain positive air pressure in a patient's airway;
(b) an air pump system comprising a plurality of pump elements and adapted to supply pressurized air to the mask; and
(c) a power supply unit connected to the air pump system by an electrical power supply line;
wherein the air pump system is mounted on one of the mask, an arm pack adapted to be worn on the patient's arm, a cap or headpiece adapted to be worn on the patient's head, a vest adapted to be worn on the patient's torso, and a waist pack adapted to be worn around the patient's waist, and wherein the power supply unit is mounted on one of the arm pack, the cap or headpiece, the vest, and the waist pack.

40. An apparatus for the supply of pressurized air to a mask comprising
(a) an air pump system comprising a plurality of pump elements adapted to supply pressurized air to the mask; and
(b) means for coupling the air pump system to the mask and for decoupling the integrated air pump system from the mask.

41. The apparatus of Claim 40 wherein the air pump system comprises 4 to 100 individual pump elements.

42. The apparatus of Claim 40 wherein the air pump system comprises 100 to 1,000 individual pump elements.

43. The apparatus of Claim 40 wherein the air pump system comprises greater than 1,000 individual pump elements.

44. The apparatus of Claim 40 which further comprises a power supply system adapted to provide power to drive the pump elements.

45. A docking station for a positive airway pressure therapy device consisting essentially of
(a) means for coupling and uncoupling the docking station and the device;
(b) means for setting operating parameters for the device and means for transferring the operating parameters to the device;
(c) means for downloading, operating data from the device to the docking station; and
(d) means for connecting the docking station to a source of electric power.

46. The docking station of Claim 45 wherein the means for coupling and uncoupling the docking station and the device includes means for connecting and disconnecting an electrical power connector between the docking station and the device.

47. The docking station of Claim 46 wherein the docking station further comprises recharging means adapted to recharge a rechargeable power supply in the device when the device is coupled with the docking station.

48. A method for providing positive airway pressure comprising
(a) providing an apparatus including
(1) a mask for the delivery of pressurized air to maintain positive airway pressure in a patient's airway; and
(2) an air pump system comprising a plurality of pump elements adapted to supply pressurized air to the mask, wherein the air pump system is attached directly to the mask or is adapted to be worn on a part of the patient's body spaced apart from the mask;
(b) mounting the mask on the patient's face; and
(c) compressing atmospheric air in the air pump system to provide pressurized air, introducing the pressurized air into the mask, and maintaining the positive airway pressure in the patient's airway during both inhalation and exhalation.

49. The method of Claim 48 wherein the pressure of the pressurized air in the mask during inhalation and exhalation is controlled at a pressure between 4 and 25 cm water.

50. The method of Claim 48 wherein the pressure of the pressurized air in the mask is controlled at a first pressure during inhalation and is controlled at a second pressure during exhalation, wherein the second pressure is less than the first pressure.

51. The method of Claim 50 wherein the first and second pressures are between 4 and 25 cm water.

52. The method of Claim 48 which further comprises providing power to operate the air pump system by an electrical supply line from an external source.

53. The method of Claim 48 which further comprises providing power to operate the air pump system by an electrical supply line from a rechargeable power source worn on the patient's body.
